# EUROPÄISCHE PATENTSCHRIFT

(11) **EP 1 377 675 B1**
(45) Veröffentlichungstag und Bekanntmachung des Hinweises auf die Patenterteilung: **22.08.2007**
(21) Anmeldenummer: 02724264.3
(22) Anmeldetag: 30.03.2002
(51) Int. Cl.: C12P 19/04, C12P 1/00, C12M 1/04

(54) **ANORDUNG UND VERFAHREN ZUR OPTIMIERTEN NÄHRSTOFF- UND SAUERSTOFFBEREITSTELLUNG FÜR MIKROBIELLE OBERFLÄCHENKULTUREN**
DEVICE AND METHOD FOR THE OPTIMISED PREPARATION OF NUTRIENTS AND OXYGEN FOR MICROBIAL SURFACE CULTURES
DISPOSITIF ET PROCEDE POUR LA PREPARATION OPTIMISEE DE SUBSTANCES NUTRITIVES ET D'OXYGENE POUR CULTURES DE SURFACE MICROBIENNES

(30) Priorität: 03.04.2001 DE 10116602
(43) Veröffentlichungstag der Anmeldung: 07.01.2004
(73) Patentinhaber: Forschungszentrum für Medizintechnik und Biotechnologie E.V., 99947 Bad Langensalza (DE)
(72) Erfinder: HORNUNG, Michael, 99089 Erfurt (DE); LUDWIG, Maria, 99610 Sömmerda (DE); SCHMAUDER, Hans-Peter, 07745 Jena (DE)
(74) Vertreter: Bock, Gerhard
(86) Internationale Anmeldenummer: PCT/EP2002/003535
(87) Internationale Veröffentlichungsnummer: WO 2002/081725

(56) Entgegenhaltungen:
- DE-A- 10 022 751
- DE-C- 4 438 023
- VANDAMME E J ET AL: "Improved production of bacterial cellulose and its application potential" POLYMER DEGRADATION AND STABILITY, BARKING, GB, Bd. 59, Nr. 1-3, Januar 1998 (1998-01), Seiten 93-99, XP004294361 ISSN: 0141-3910
- DATABASE CA [Online] CHEMICAL ABSTRACTS SERVICE, COLUMBUS, OHIO, US; DE BAETS, S. ET AL: "Cellulose production by Acetobacter xylinum: fermentation optimization and application potential" retrieved from STN Database accession no. 128:2946 XP002209513 & MEDEDELINGEN - FACULTEIT LANDBOUWKUNDIGE EN TOEGEPASTE BIOLOGISCHE WETENSCHAPPEN (UNIVERSITEIT GENT) (1997), 62(4A), 1231-1238 ,

## Beschreibung

Die Erfindung betrifft eine Anordnung und ein Verfahren zur optimierten Nährstoff- und Sauerstoffbereitsteflung für mikrobielle Oberflächenkulturen.

Mikrobielle Oberflächenkulturen, die auch als emerse Kulturen bezeichnet werden, sind wiederholt in der biotechnologischen Literatur beschrieben worden.

Als Beispiele hierfür sind das Oberflächenverfahren zur Essigsäureproduktion mit *Acetobacter xylinum* (Schlegel, H. G., Allgemeine Mikrobiologie, 7. überarbeitete Auflage, 1992, Thieme-Verlag, Stuttgart) und die emerse Kultivierung von *Acetobacter xylinum* zur Zelluloseherstellung (DE OS 100 22 751 A1) zu nennen.

Die Versorgung von emersen Mikroorganismenkulturen in traditionellen Flüssigmedien mit Nährstoffen, wie Kohlenstoff- und Stickstoffquellen, anorganischen Salzen und Sauerstoff bei aerobem Stoffwechsel ist selten optimal möglich. Durch die Film- bzw. Schichtbildung der Mikroorganismen, oder der durch sie gebildeten Stoffwechselprodukte, an der Grenzfläche zwischen der Nährlösung und der umgebenden Luft entsteht eine Transportbarriere sowohl für Zuführung der notwendigen Nährstoffe aus dem Medium als auch für den Abtransport der gewünschten Produkte bzw. Stoffwechselendprodukte in das Medium. Darüber hinaus entsteht durch dieses Phänomen an der Grenzfläche für die Zuführung des für aerob wachsende Mikroorganismen notwendigen Sauerstoffs ebenfalls eine Transportbarriere für die in Richtung Medium befindlichen Mikroorganismen. Dadurch kommt es in der Regel im Verlauf des Wachstums der Mikroorganismenkultur zu einer Stagnation des Wachstums und der Produktbildung oder sogar zum Absterben der aktiven Mikroorganismen. In den Lehrbüchern werden verschiedene Limitierungen der Nährstoffzufuhr beschrieben. Demnach können sich Mikrostandorte ausbilden, in denen Nährstoffkonzentrationen unterhalb einer benötigten Grenzkonzentration vorliegen und/oder verminderte O₂-Partialdrücke herrschen.

Darüber hinaus ist die Erzeugung von Aerosolen für verschiedene technische Anwendungen bekannt, wie z.B. bei der Erzeugung von dünnen Schichten (WO 0039358 A1), zur Mikroverkapselung von Materialien (EP 0927047 A2), zur Reinigung und Aufarbeitung von Materialien und Naturstoffen (EP 0839586 A2, DE 4420730 A1, FR 9509196), zur Pulverisierung (WO 9717933 A1) und beim Einsatz in der Medizin (bspw. DE OS 4003989 A1).

In der Pflanzenzüchtung werden bspw. Lösungen versprüht (EP 4124672 A1, EP 0004103 A1), Aerosole in hydroponischen Kultursystemen genutzt (EP 02476527 A1) und Kulturlösungen, die das Wachstum von Pflanzen stimulieren, vernebelt (WO 9734487 A1, EP 0792693 A1, EP 0295352 A2).

Auch in der Biotechnologie sind Anwendungen von Aerosolen beschrieben, z.B. zur Desodorierung und Stabilisierung biotechnologisch gewonnener Wertstoffe und deren wässriger Zubereitungen (EP 0765182 A1) und zur Einzellerproteinherstellung (DE 3239210 C2). Als weiteres Einsatzgebiet wird der Transfer von DNA und Makromolekülen im Rahmen der Gentechnik genannt (EP 0482125 A1).

Die Anwendung von Substrataerosol im Zusammenhang mit einem Verfahren zur Substratversorgung bei der Kultivierung von Mikroorganismen in einem aeroben Fermentationsprozess ist aus der Patentschrift DE 4438023 C1 bekannt. Bei dieser Anwendung werden die Substrate in einem Fermenter mit submers wachsenden Mikroorganismen zur Verbesserung der Nährstoffverfügbarkeit fein verteilt in Form eines Aerosols in das Kulturmedium eingebracht.
Eine Applizierbarkeit dieses Verfahrens für nicht frei in der Fermenterlösung vorhandene Mikroorganismen (emerse Kultur) ist in der Schrift nicht offenbart.

Zur Erzeugung von Aerosolen werden unterschiedliche physikalische Wirkprinzipien ausgenutzt die u.a. in den Schriften DE 4134665 A1, DE 4305277 A1, DE 4438292 A1, DE 19523648 A1, DE 19838711 C1, WO 97/34487 A1, DE 4422710 C1, DE 19726110 A1 beschrieben werden.

Durch die Erfindung sollen die im Zusammenhang mit der Versorgung von emersen Mikroorganismenkulturen aufgezeigten Nachteile bzgl. der Nährstoff- und Sauerstoffbereitstellung vermieden werden.
Der Erfindung liegt die Aufgabe zugrunde, unter besonderer Berücksichtigung der Wachstumslimitation der kultivierten Mikroorganismen, hervorgerufen z.B. durch die Bildung von Festprodukten (wie bspw. Zellulose) durch eine optimierte Nährstoffbereitstellung diese Limitation zu überwinden, so dass eine optimale Nährstoff- und Sauerstoffversorgung von Mikroorganismen emerser Kulturen zur Maximierung der Produktausbeute führt, um je nach Art der Kultur bspw. die Herstellung homogener Schichten von Zellulose nicht limitierter Schichtdicke oder eine höhere Konzentration von gelösten Zielprodukten zu erreichen.

Gemäß der Erfindung wird diese Aufgabe durch die kennzeichnenden Merkmale des ersten und siebenten Patentanspruchs gelöst. Vorteilhafte Ausgestaltungen werden durch die untergeordneten Ansprüche 2 bis 6 und 8 bis 9 angegeben.

Das Wesen der Erfindung besteht darin, dass mittels der erfindungsgemäßen Anordnung und des erfindungsgemäßen Verfahrens die sich in einer Grenzschicht, d.h. in dem Luft- und Nährlösungsraum entlang einer Grenzfläche befindlichen aktiven, emers wachsenden Mikroorganismen durch eine Nährstoffzufuhr mit Hilfe eines Aerosols direkt auf die Oberfläche der Emerskultur versorgt werden, um so die Transporthemmungen, z.B. hervorgerufen durch ihre eigene Myceldecke oder die gebildeten Produkte, wie bspw. Zellulose, auszuschließen, ohne dass eine mechanische Beeinträchtigung der Kulturen erfolgt.
Dadurch werden die Probleme, die beim Stand der Technik bzgl. emerser Kultivierungen in Hinblick auf die Nährstoff- und Sauerstoffverfügbarkeit vorhanden sind, eliminiert, so dass ausreichend Nährstoffe sowie optimale Sauerstoffmengen für den aeroben Stoffwechsel der emers wachsenden Mikroorganismen bereitgestellt werden können.

Die Erfindung wird nachstehend an Hand der Zeichnungen und der Ausführungsbeispiele näher erläutert. Es zeigt:
- Fig. 1:: eine Ausführungsform der erfindungsgemäße Anordnung in schematischer Darstellung,
- Fig. 2:: einen Bestandteil der erfindungsgemäße Anordnung in der Aufsicht,
- Fig. 3:: den in Fig. 2 dargestellten Bestandteil und einen weiteren Bestandteil der erfindungsgemäße Anordnung in einer schematischen Funktionsdarstellung.

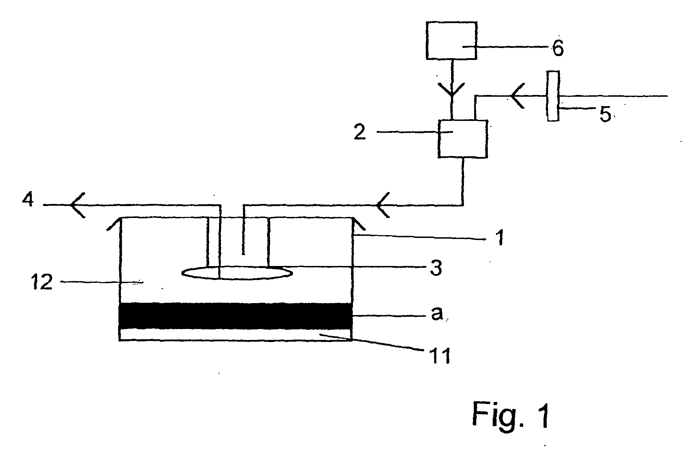

Wie in Fig. 1 dargestellt, besteht die Anordnung zur optimierten Nährstoff und Sauerstoffbereitstellung für mikrobielle Oberflächenkulturen aus einem Kulturgefäss 1, einem Substrataerosolerzeuger 2, einem Substrataerosolverteiler 3 und einer Substrataerosolabführung 4.
Das Kulturgefäss 1 umschließt dichtend einen Nährlösungsraum 11 und einen Luftraum 12. Es kann mit einem Deckel verschlossen sein und erfüllt die im biotechnologischen Apparatebau üblichen Anforderungen wie Sterilisierbarkeit, Totraumfreiheit, Konstanthaltung einer gewünschten Prozesstemperatur und es ist problemlos zu reinigen. Die Fertigung des Kulturgefässes 1 erfolgt bspw. aus Glasscheiben, die mit temperaturstabilem Klebstoff verbunden sind oder aus Edelstahl. Zwischen Kulturgefäss und Abdeckung ist ein Dichtungssystem vorhanden, dass das Eindringen von Fremdkeimen aus der Umgebung sicher verhindert.
Der Nährlösungsraum 11 ist mit einer Nährlösung und emers wachsenden Mikroorganismen befüllbar, wobei der Nährlösungsraum 11 über eine Grenzfläche a mit einem Luftraum 12 verbunden und die Grenzfläche a zwischen dem Boden - und dem Deckel des Kulturgefässes 1 anordenbar ist.

Der Substrataerosolerzeuger 2, der Substrataerosolverteiler 3 und die Substrataerosolabführung 4 sind in dem Luftraum 12 über der Grenzfläche a angeordnet und mit Luft sowie Nährlösung durchströmbar.

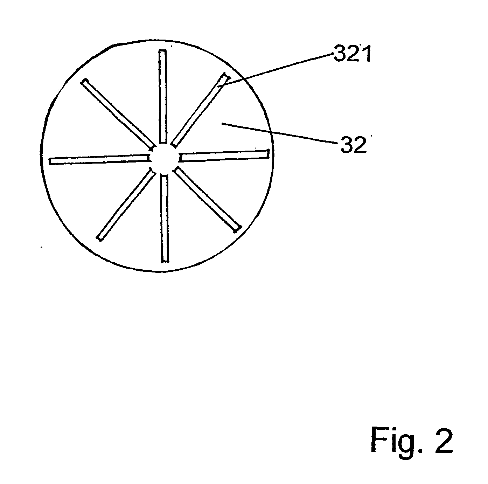

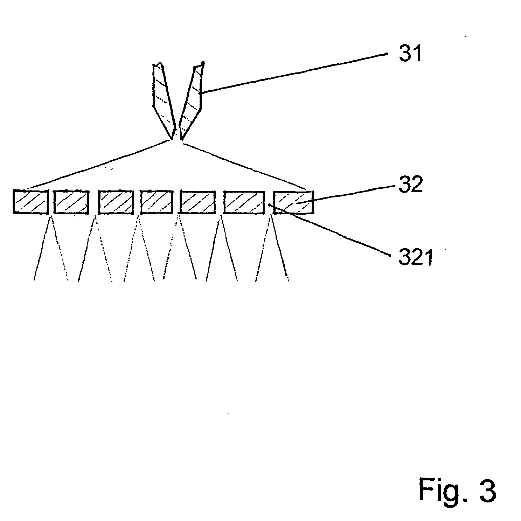

Der Substrataerosolverteiler 3 besteht wie in Fig. 2 dargestellt, aus einer Düse 31 und einem Schlitzverteiler 32. Der Schlitzverteiler 32 ist zwischen der Düse 31 und der Grenzfläche a angeordnet und besteht bspw., wie in Fig. 3 dargestellt, aus einem äußeren Rahmen, in den eine abgewinkelte Schlitzplatte fest eingebracht ist.

Der Substrataerosolerzeuger 2 ist mit einem Sterilfilter 5 und mit einem Nährlösungsvorratsgefäss 6 verbunden.

Die Nährstofferosolerzeugung erfolgt optimal mittels Ultraschallschwingungen innerhalb des Substrataerosolaerzeugers 2, der bspw. zylindrisch sein kann. In den Substrataerosolerzeuger 2 ist ein Ultraschallwandler gemäß dem Stand der Technik integriert. Die Ultraschallschwingungen werden von einem Generator erzeugt, der räumlich vom Schallwandler getrennt ist und über eine elektrische Steckverbindung mit diesem verbunden ist. In die Aerosolkammer wird die flüssige Nährlösung aus dem Nährlösungsvorratsgefäss 6 eingebracht. Dabei kann die Vernebelung der Nährlösung bspw. aus einem stehenden Flüssigkeitslevel über dem Schallwandler heraus erfolgen oder die Nährlösung wird über eine Kanüle tropfenweise direkt auf den Ultraschallwandler dosiert. ,
Die Tröpfchendurchmesser nach der Ultraschallbehandlung sollte erfindungsgemäß im Bereich von 0,1-15µm, vorzugsweise im Bereich von 0,5-6 µm liegen.
Während des Ultraschallbetriebes wird der Substrataerosolerzeuger 2 mit Nährlösungsaerosol gefüllt. Durch einen Luftstrom, der bspw. von einem Ventilator erzeugt werden kann, wird das Nährlösungsaerosol, bspw. über eine flexible Schlauchverbindung, zum Substrataerosolverteiler 3 transportiert. Dabei lässt sich die Aerosolkonzentration über die Intensität der Ultraschallschwingungen und über die Ventilatordrehzahl stufenlos variieren. Die Frequenz und die Dauer der Dosierintervalle können bspw. über eine zeitliche Steuerung vorgegeben werden.

Alle Teile des Substrataerosolverteilers 3, die mit Nährlösung in Kontakt kommen, sind bei ca. 134°C autoklavierbar bzw. bei ca. 180°C hitzesterilisierbar. Der Luftstrom wird durch den Sterilfilter 5, der im Beispiel eine Porenweite von 0,2 µm besitzt, von Keimen befreit.

Der Substrataerosolverteiler 3 kann, wie im Beispiel der Fig. 1, in den Deckel des Kulturgefässes 1 integriert sein. Der Substrataerosolverteiler 3 stellt sicher, dass das Nährlösungsaerosol gleichmäßig über die gesamte Grenzfläche a innerhalb des Kulturgefässes 1 verteilt wird.
Durch die Substrataerosolabführung 4, die im Beispiel, wie der Substrataerosolverteiler 3, durch den Deckel des Kulturgefässes 1 gehaltert ist, wird die an dem Deckel des Kulturgefässes 1 kondensierte Nährlösung aufgefangen und abgeführt, so dass die Oberfläche der Kultur vor dem Auftreffen großer Tropfen geschützt ist.
Dies ist bspw. besonders wichtig für die Herstellung bakterieller Zellulose, deren Homogenität aufgrund auftropfender, großer Flüssigkeitstropfen im Bereich der Grenzfläche a gestört werden würde, was zu einer inhomogenen und fehlerhaften Zelluloseschicht führen würde.
Durch die Düse 31 oder mehrere Düsen 32 wird das Nährstoffaerosol an einem oder mehreren Punkten entlang der Längsachse des Schlitzverteilers 32 zudosiert. Dadurch wird das Volumen zwischen dem Schlitzverteiler 32 und dem Deckel des Kulturgefässes 1 mit Aerosol gefüllt. Gleichzeitig gelangt das Aerosol durch die Ausnehmungen 321 des Schlitzverteiles 32 in den unteren Bereich des Kulturgefässes 1. Durch die flächendeckende Anordnung der Ausnehmungen 321 über dem Gefäss wird das Nährlösungsaerosol über die Grenzfläche a des Kulturgefässes 1 homogen verteilt. Während dieser Vernebelung entstehendes Kondensat wird über die Substrataerosolabführung 4 abgeführt.
Diese abgeführte, kondensierte Nährlösung ist nach erfolgter Sterilisation in das Nährlösungsvorratsgefäss 6 rückführbar.

Ebenso wirkungsvoll wie der Schlitzverteiler 32 ist ein Ventilator bzw. ein System aus mehreren Ventilatoren unterhalb einer abgewinkelten bzw. konvex gewölbten Abdeckung des Kulturgefässes (nicht als Fig. dargestellt). Die Ventilatoren werden über Magnetkupplungen unter sterilen Bedingungen angetrieben. Die Ventilatoranzahl, -drehzahl und
- form sind so ausgewählt, dass eine homogene Verteilung des Nährlösungsaerosols erfolgt.

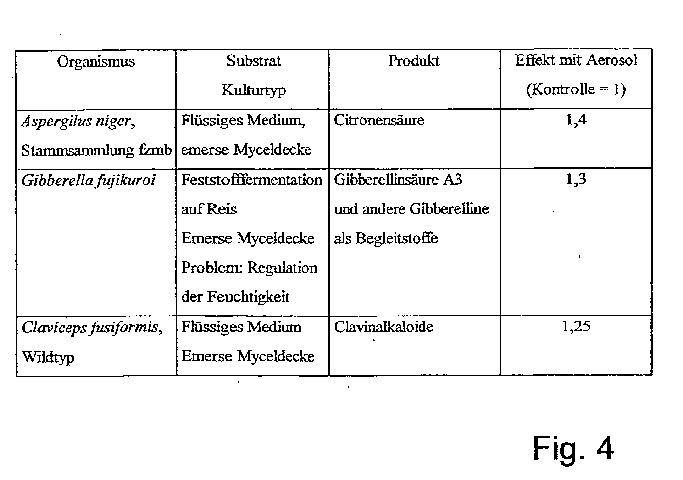

Nachfolgend wird die Erfindung am Beispiel der Bildung von Zelluloseschichten durch *Glucoacetobacter xylinum* beschrieben und in einem zweiten Beispiel zur Produktbildung in Fig. 4 tabellarisch dargestellt.
In dem ersten Beispiel erfolgt die Zellulosebildung durch *Glucoacetobacter xylinum.* Es ist bekannt, dass sich bakterielle Zellulose (BC) durch emerse Kultivierung von z.B. *Glucoacetobacter xylinum* bilden lässt. Eine einfache Möglichkeit zur biotechnologischen Produktion von BC stellt die Kultivierung in aquarienähnlichen Gefässen dar. Diese Gefässe werden unter sterilen Bedingungen mit einem optimierten Nährmedium gefüllt und mit einer Bakteriensuspension (DE 100 22 751 A1) beimpft. Zum Schutz vor Fremdinfekten werden die Kulturgefässe dicht verschlossen und in Brutschränken unbewegt inkubiert. Bei Temperaturen von 30°C setzt nach 2 bis 3 Tagen eine sichtbare Zelluloseproduktion ein. Aus anfangs inselartigen Zellulosefragmenten auf der Flüssigkeitsoberfläche bildet sich eine zusammenhängende Zelluloseschicht. Die Schichtdicke nimmt zu und erreicht innerhalb von 4 bis 6 Wochen eine Schichtdicke von 3 bis 6 cm. Nach Ablauf dieser Kultivierungszeit stagniert die Zelluloseproduktion, die Dicke der Zelluloseschicht bleibt konstant. Die Ursache für diese Stagnation ist die Limitierung des Nährstoffangebotes, das den zellulosebildenden Bakterien zur Verfügung steht. Lebensfähige Zellulosebildner befinden sich nur auf der Oberfläche der Zelluloseschicht. Die Nährlösung befindet sich unterhalb der Schicht. Ab einer Schichtdicke von 3 bis 6 cm ist die Transportbarriere so groß, dass die Substrate der Nährlösung nicht mehr bis zur Oberfläche der Zelluloseschicht gelangen und den dort anhaftenden Bakterien als Kohlenstoffquelle dienen kann. Ab diesem Zeitpunkt muss in herkömmlichen emersen Kultivierungen die Inkubation abgebrochen und die Zellulose geerntet werden.

An diesem Punkt setzt die erfindungsgemäße Anordnung und das erfindungsgemäße Verfahren an.
Erfindungsgemäß werden die aktiven Zellulosebildner nicht ausschließlich mit Substraten aus dem Nährlösungsraum unterhalb der Zelluloseschicht versorgt, sondern zusätzlich mit Nährlösungsaerosol beaufschlagt. Dadurch werden Inhibitionen, verursacht durch die Transportwege, ausgeschlossen.
Der Substrataerosolerzeuger der zuvor beschriebenen und in Fig. 1 dargestellten Anordnung wird mit einer Ultraschallfrequenz von ca. 1,7 MHz betrieben. Daraus resultiert eine mittlere Tröpfchendurchmesser von 0,5 - 6 µm. Bei einem eingestellten Luftvolumenstrom von 5-10 1/min werden dabei bis zu 4 ml Nährlösung pro Minute vernebelt. Die Dauer der Dosierintervalle beträgt 10 min in stündlichem Rhythmus. Das Aerosol wird mit Hilfe des Substrataerosolverteilers über die Wachstums-und Grenzfläche von ca. 600 cm² gleichmäßig verteilt. Die aerobe Bakterienkultur wird über einen Zeitraum von 4 Wochen oder mehr bei 30°C inkubiert, wobei sich eine kompakte Zelluloseschicht ohne Inhomogenitäten bildet. Die Schichtdickenzunahme ist dabei zeitproportional. Bei Gewährleistung der Sterilität des gesamten Systems könnten durch die erfindungsgemäße Anordnung und das erfindungsgemäße Verfahren Zelluloseschichtdicken von 10 cm und mehr erreicht werden.

Die in Fig. 4 zusammengefassten Ergebnisse zeigen, dass es möglich ist, auch andere emerse Kulturen, wie Feststofffermentationen in ihrem Ablauf durch die erfindungsgemäße Beaufschlagung mit Nährstoffaerosolen bzw. Aerosolen günstiger zu fermentieren.

### Bezugszeichenliste

- 1 -: Kulturgefäss
- 11 -: Nährlösung
- 12 -: Luft
- 2 -: Substrataerosolerzeuger
- 3 -: Substrataerosolverteiler
- 31 -: Düse
- 32 -: Schlitzverteiler
- 321 -: Ausnehmungen
- 4 -: Substrataerosolabführung
- 5 -: Sterilfilter
- 6 -: Nährlösungsvorratsgefäss
- a -: Grenzfläche

## Patentansprüche

1. Anordnung zur optimierten Nährstoff- und Sauerstoffbereitstellung für mikrobielle Oberflächenkulturen bestehend aus einem, einen Nährlösungsraum(11) und einen Luftraum (12) umschließenden Kulturgefäss (1), einem Substrataerosolerzeuger (2), einem Substrataerosolverteiler (3) und einer Substrataerosolabführung (4), wobei
der Substrataerosolerzeuger (2), der Substrataerosolverteiler (3) und die Substrataerosolabführung (4) in dem Luftraum (12), der über eine Grenzfläche (a) mit einem Nährlösungsraum (11) verbunden ist, angeordnet sind,
die Grenzfläche (a) zwischen der Bodenregion - und Deckelregion des Kulturgefässes (1) anordenbar ist und
der Substrataerosolerzeuger (2) auf der, der Grenzfläche (a) abgewandten Seite des Substrataerosolverteilers (3), welcher sich zwischen der Grenzfläche (a) und der Deckelregion des Kulturgefässes (1) befindet, angeordnet ist.

2. Anordnung nach Anspruch 1, **dadurch gekennzeichnet, dass** der Substrataerosolverteiler (3) aus einer Düse (31) und einem Schlitzverteiler (32) besteht, wobei der Schlitzverteiler (32) zwischen der Düse (31) und der Grenzfläche (a) angeordnet ist.

3. Anordnung nach Anspruch 1, **dadurch gekennzeichnet, dass** der Substrataerosolerzeuger (2) mit einem Sterilfilter (5) verbunden ist.

4. Anordnung nach Anspruch 1, **dadurch gekennzeichnet, dass** der Substrataerosolerzeuger (2) mit einem Nährlösungsvorratsgefäss (6) verbunden ist.

5. Anordnung nach den Ansprüchen 1 bis 4, **dadurch gekennzeichnet dass** der Nährlösungsraum (11) mit Nährlösung und emers wachsenden Mikroorganismen befüllbar ist.

6. Anordnung nach den Ansprüchen 1 bis 4, **dadurch gekennzeichnet, dass** der Substrataerosolerzeuger (2) Substrataerosolverteiler (3) und die Substrataerosolabführung (4) mit Luft und Nährlösung durchströmbar ist.

7. Verfahren zur optimierten Nährstoff- und Sauerstoffbereitstellung für mikrobielle Oberflächenkulturen, **dadurch gekennzeichnet, dass** eine Anordnung gemäß Anspruch 1 verwendet wird, indem Nährlösung aus dem Nährlösungsvorratsgefäss (6) und Luft, den Sterilfilter (5) passierend, durch den Substrataerosolerzeuger (2) hindurch, über die Düse (31), durch den Schlitzverteiler (32) hindurch und an diesem vorbei in dem Luftraum (12) über der Grenzfläche (a) vernebelt und der überschüssige Nebel über die Substrataerosolabführung (4) abgesaugt wird, wobei sich emers wachsende Mikroorganismen im Nährlösungsraum (11) entlang der Grenzfläche (a) befinden.

8. Verfahren nach Anspruch 7, **dadurch gekennzeichnet, dass** die emers wachsenden Mikroorganismen Zellulosebildner sind und entlang der Grenzfläche (a) homogene Schichten von Zellulose synthetisieren.

9. Verfahren nach Anspruch 7, **dadurch gekennzeichnet, dass** die emers wachsenden Mikroorganismen Mycelbildner sind und entlang der Grenzfläche (a) Stoffwechselprodukte in den Nährlösungsraum (11) ausscheiden.

## Claims

1. Device for the optimised preparation of nutrients and oxygen for microbial surface cultures with said device consisting of a culture vessel (1), which encloses a nutrient solution chamber (11) and an air chamber (12), of a substrate aerosol producer (2), of a substrate aerosol distributor (3) and of a substrate aerosol evacuating device (4),
with said substrate aerosol producer (2), substrate aerosol distributor (3) and substrate aerosol evacuating device (4) being arranged in the air chamber (12) that is connected with a nutrient solution chamber (11) by means of a boundary surface (a), and
said boundary surface (a) can be arranged between the bottom region and the covering region of the culture vessel (1), and
the substrate aerosol producer (2) is arranged on the side of the substrate aerosol distributor (3) that is turned away from the boundary surface (a), said substrate aerosol distributor (3) being situated between the boundary surface (a) and the covering region of the culture vessel (1).

2. Arrangement according to claim 1, wherein the substrate aerosol distributor (3) consists of a nozzle (31) and a slot distributor (32) with the slot distributor (32) being arranged between the nozzle (31) and the boundary surface (a).

3. Arrangement according to claim 1, wherein the substrate aerosol producer (2) is connected with a sterile filter (5).

4. Arrangement according to claim 1, wherein the substrate aerosol producer (2) is connected with a nutrient solution supply vessel (6).

5. Arrangement according to claims 1 through 4, wherein the nutrient solution chamber (11) can be filled with nutrient solution and micro-organisms growing in an emerged manner.

6. Arrangement according to claims 1 through 4, wherein air and nutrient solution can flow through the substrate aerosol producer (2), the substrate aerosol distributor (3) and the substrate aerosol evacuating device (4).

7. Method for the optimised preparation of nutrients and oxygen for microbial surface cultures, wherein an arrangement according to claim 1 is used to pass the nutrient solution from the nutrient solution supply vessel (6) and air through the sterile filter, then through the substrate aerosol producer (2) via the nozzle (31) and through the slot distributor (32),said mixture being sprayed past the same into the air chamber (12) over the boundary surface (a), and the excess spray is absorbed by means of the substrate aerosol evacuating device (4), and micro-organisms growing in an emerged manner are located in the nutrient solution chamber (11) along the boundary surface (a).

8. Method according to claim 7, wherein the micro-organisms growing in an emerged manner are cellulose producers and synthesise homogeneous layers of cellulose along the boundary surface (a).

9. Method according to claim 7, wherein the micro-organisms growing in an emerged manner are mycelium producers and set metabolic products free in the nutrient solution chamber (11) along the boundary surface (a).

## Revendications

1. La configuration de la mise à disposition optimisée de substances nutritives et de l'oxygène pour les cultures microbiennes de surface, composée d'un récipient de culture (1) comprenant un compartiment pour la solution nutritive (11) et un compartiment à air (12), ainsi que d'un générateur d'un substrat aérosol (2), d'un diffuseur du substrat aérosol (3) et d'un dispositif d'évacuation du substrat aérosol (4),
est **caractérisée en ce que** le générateur du substrat aérosol (2), le diffuseur du substrat aérosol (3) et le dispositif d'évacuation du substrat aérosol (4) sont disposés dans le compartiment à air (12) relié par une aire interfaciale (a) au compartiment contenant la solution nutritive (11),
que l'aire interfaciale (a) peut être située entre la zone du fond et celle du couvercle du récipient de culture et
que le générateur du substrat aérosol (2) est disposé sur le côté opposé à l'aire interfaciale (a) du diffuseur du substrat aérosol (3), celui-ci étant positionné entre l'aire interfaciale (a) et la zone au niveau du couvercle du récipient de culture (1).

2. La configuration selon la revendication 1 est **caractérisée en ce que** le diffuseur du substrat aérosol (3) se compose d'une buse (31) et d'un diffuseur à fente (32) disposé entre la buse (31) et l'aire interfaciale (a).

3. La configuration selon la revendication 1 est **caractérisée en ce que** le générateur du substrat aérosol (2) est relié à un filtre stérile (5).

4. La configuration selon la revendication 1 est **caractérisée en ce que** le générateur du substrat aérosol (2) est relié à un réservoir de solution nutritive (6).

5. La configuration selon les revendication 1 à 4 est **caractérisée en ce que** le compartiment de la solution nutritive (11) peut être rempli d'une solution nutritive et de microorganismes se développant à la surface.

6. La configuration selon les revendication 1 à 4 est **caractérisée en ce que** le l'air et la solution nutritive peuvent passer par le générateur du substrat aérosol (2), le diffuseur du substrat aérosol (3) et le dispositif d'évacuation du substrat aérosol (4).

7. Le procédé pour la préparation optimisée des substances nutritives et de l'oxygène pour cultures de surface microbiennes est **caractérisé en ce qu'**une configuration selon la revendication 1 est appliquée de sorte que la solution nutritive provenant du réservoir de solution nutritive (6), ainsi que l'air, passent par le filtre stérile (5), le générateur du substrat aérosol (2), la buse (31) et le diffuseur à fente (32) ainsi qu'à côté de celui-ci, pour être enfin pulvérisé dans le compartiment à air (12) au-dessus de l'aire interfaciale (a), l'excédent du substrat pulvérisé se trouvant évacué par aspiration moyennant un dispositif assurant l'évacuation du substrat aérosol (4); des microorganismes se développent donc dans le compartiment de la solution nutritive (11), le long de l'aire interfaciale (a) à la surface.

8. Le procédé selon la revendication 7 est **caractérisé en ce que** les microorganismes se développant à la surface produisent de la cellulose et synthétisent des couches homogènes de cellulose le long de l'aire interfaciale (a).

9. Le procédé selon la revendication 7 est **caractérisé en ce que** les microorganismes qui se développent à la surface produisent un tissu mycélien et secrètent des substances métaboliques dans le compartiment contenant la solution nutritive (11).
